# EUROPEAN PATENT APPLICATION

(11) **EP 1 118 658 A1**
(43) Date of publication of application: **25.07.2001**
(21) Application number: 99970116.2
(22) Date of filing: 01.10.1999
(51) Int. Cl.: C12N 1/00

(54) **METHOD FOR PREPARING HIGH PERFORMANCE RecA-LIKE RECOMBINASE/SINGLE-STRANDED NUCLEIC ACID PROBE COMPLEX AND UTILIZATION THEREOF**

(30) Priority: 01.10.1998 JP 28038098
(71) Applicant: Daikin Industries, Ltd., Osaka-shi, Osaka 530-8323 (JP)
(72) Inventor: KIGAWA, Koji, Daikin Industries, Ltd., Settsu-shi, Osaka 566-8585 (JP); KUSUMI, Kayo, Daikin Industries, Ltd., Settsu-shi, Osaka 566-8585 (JP); MUKAI, Eli, Daikin Industries, Ltd., Settsu-shi, Osaka 566-8585 (JP); OBATA, Kazuaki, Daikin Industries, Ltd., Settsu-shi, Osaka 566-8585 (JP)
(74) Representative: Grünecker, Kinkeldey, Stockmair & Schwanhäusser Anwaltssozietät
(86) International application number: JP9905420
(87) International publication number: WO0020558

(57) **Abstract**

It is possible to form a RecA-like recombinase/single-stranded nucleic acid probe/double-stranded target nucleic acid complex very efficiently and specifically, by preparing the RecA-like recombinase/single-stranded nucleic acid probe complex in the presence of a nonhydrolyzable nucleotide co-factor whose molecule number is one quarter or more of the number of molecules of nucleotide residues contained in the single-stranded nucleic acid probe and 10 times or less the number of RecA-like recombinase molecules, and then contacting the complex with a sample containing double-stranded target nucleic acid.

## Description

### Technical Field

The present invention relates to a method for preparing a RecA-like recombinase/single-stranded nucleic acid probe complex (single-stranded nucleoprotein filament) and utilization of the complex.

### Background Art

A variety of recombinases, which catalyze in *vitro* homologous pairing and/or DNA-strand exchange, have been isolated and purified from various prokaryotes and eukaryotes. Among these recombinases, the RecA protein, a recombinase derived from *Escherichia coli,* has been extensively investigated (Shibata, T., Cell Technology, 1990, 9(4), 281-292). The RecA protein is known to catalyze in *vitro* homologous pairing of single-stranded DNA with double-stranded DNA and thus generate homologously-paired triple-stranded DNA or other triple-stranded joint DNA molecules (Rigas, B. et al., Proc. Natl. Acad. Sci. USA, 1986, 83, 9591-9595; Hsieh, P. et al., Proc. Natl. Acad. Sci. USA, 1992, 89, 6492-6496; Ferrin, L. J. et al., Science, 1991, 254, 1494-1497; etc.). The RecA protein is also known to form a four-stranded DNA structure called a double D-loop between two types of single-stranded DNA complementary to each other (complementary single-stranded DNA), and a double-stranded DNA which has a homologous site for the single-stranded DNA (Sena, E. P., Nature Genetics, 1993, 3, 365-372; Jayasena, V. K. et al., J. Mol. Biol., 1993, 230, 1015). In addition to pairing between DNA and DNA, the RecA protein is also known to pair DNA with its homologous RNA (Kirkpatrick, D. et al., Nucleic Acids Res., 1992, 20, 4339-4346 and 4347-4353), and with its homologous RNA/DNA hybrid (Kotani, H. et al., Mol. Gen. Genet., 1996, 250, 626-634). By utilizing such properties of the RecA protein, methods for isolating specific double-stranded target DNA existing within solutions in very small amounts (in molar ratios of 1:50 molecules to 1:several hundred molecules) have been developed (Honigberg, S.M. et al., Proc. Natl. Acad. Sci. USA, 1986, 83, 9586-9590; Rigas, B. et al., Proc. Natl. Acad. Sci. USA, 1986, 83, 9591-9595; Teintze M. et al., Biochem. Biophys. Res. Commun., 1995, 211, 804-811; US Patent No. 4,888,274). In situ hybridization methods have also been developed for detecting double-stranded target DNA in fixed cells (WO93/05177).

However, in order to target, enrich, detect, and/or isolate extremely small amounts (for example, a molar ratio of one molecule or less to 1,000 molecules) of double-stranded target nucleic acid molecules in a sample by utilizing recombinases represented by the RecA protein, it is essential to develop a method for preparing a more suitable and high-performance RecA-like recombinase/single-stranded nucleic acid probe (containing a homologous probe having a sequence sufficiently complementary to the double-stranded target nucleic acid sequence) complex (single-stranded nucleoprotein filament) and enhance the reaction efficiency, yield, and specificity.

### Disclosure of the Invention

An objective of the present invention is to provide a method for preparing a RecA-like recombinase/single-stranded nucleic acid probe (containing a homologous probe having a sequence sufficiently complementary to the double-stranded target nucleic acid sequence) complex having a high reactivity and specificity to double-stranded target nucleic acid in a sample. Another objective is to provide the utilization of the RecA-like recombinase/single―stranded nucleic acid probe complex prepared by the method of the invention for targeting, enriching, detecting, and/or isolating double-stranded target nucleic acid sequence.

Radding et al. (US Patent No. 4,888,274) have developed a method for preparing a stable RecA/single-stranded nucleic acid probe complex (single-stranded nucleoprotein filament) capable of effectively and specifically forming a stable complex with a double-stranded target nucleic acid having a complementary sequence. In this method, a single-stranded DNA is allowed to react with the RecA protein in the presence of ATPγS and a low concentration of Mg²⁺, but in the absence of a single-strand binding protein (SSB). Radding et al. describe that it is preferable to conduct the reaction in the presence of 0.5 to 2 mM ATPγS, and that at least 0.5 mM or more is needed (see page 7 and claim 8 in the same patent).

Basically, one molecule of RecA protein is thought to bind to one molecule of a nucleotide co-factor such as ATP. When used as the co-factor, ATP is hydrolyzed to ADP by the ATPase activity of the RecA protein itself. The resulting RecA protein/ADP complex dissociates from single-stranded DNA. Therefore, it is necessary to use a large excess of ATP or an ATP-regeneration system (such as phosphocreatine + creatine kinase, etc.) for the formation of a stable RecA/single-stranded nucleic acid probe complex. However, in the case of a co-factor such as ATPγS, which is hardly hydrolyzed by ATPase, the co-factor is not hydrolyzed to ADP, and thus, it does not dissociate from the single-stranded nucleic acid via the formation of the RecA protein/ADP complex, and the affinity for RecA protein is extremely high. Therefore, the present inventors thought that it was unnecessary to add a large excess of the co-factor. From this point of view, in case of using a nonhydrolyzable nucleotide co-factor (such as ATPγS), the present inventors further thought that what is important is not the absolute concentration of such co-factor present within the reaction solution, but how many molecules of the co-factor per nucleotide residue constituting the single-stranded nucleic acid probe and per RecA protein should be optimal within the reaction solution.

In US Patent No. 4,888,274, Radding et al. describe that the formation of a stable RecA/single-stranded DNA probe complex, which is capable of effectively and specifically forming a stable complex with the double-stranded target DNA having a complementary sequence, can be carried out preferably in the presence of 0.5 to 2 mM Mg²⁺, 0.5 to 2 mM ATPγS, 0.1 to 50 µM single-stranded DNA (in this case, the number of moles means the number of moles of nucleotide residue in the DNA, and 1 M = 350 g/l), and RecA protein whose molar amount is one quarter or more, preferably one third (about 0.033 to 16.7 µM), of the molar amount of single-stranded DNA (pages 7 to 8 in the same patent). In this case, the number of ATPγS molecules corresponds to 10 to 20,000 times the number of molecules of nucleotide residues constituting single-stranded DNA, and to about 30 to 60,000 times the number of RecA protein molecules. The patent also describes that it is necessary to optimize the amount of RecA, depending upon the amount of total DNA (probe + double-stranded DNA) in the sample, in order to efficiently form a nucleotide sequence-specific probe/RecA/double-stranded target DNA complex by using the RecA/single-stranded DNA probe complex (pages 9 to 10 in the same patent). Further stated is that the optimal RecA concentration was 0.32 µM, and a lower degree of formation of the probe/RecA/double-stranded target DNA complex was seen at a RecA concentration less than 0.16 µM, or higher than 0.64 µM, based on the result of an experiment where RecA concentration was altered from 0.08 to 5.12 µM in the presence of 1.6 mM ATPγS, 8 fmol of single-stranded probe (in 100 µl; this amount corresponds to about 0.46 µM in molar concentration of nucleotide residue; lines 43 to 49, page 10 in the same patent), and 0.32 nM double-stranded DNA. Therefore, in this case, the number of ATPγS molecules corresponds to about 3,500 times the number of molecule of nucleotide residues in the single-stranded probe, and 2,500 to 10,000 times the number of RecA protein molecules.

As described above, generally, a large excess of nonhydrolyzable nucleotide co-factors such as ATPγS (at least 10 times the number of molecules of nucleotide residues in the single-stranded probe and 30 times the number of RecA protein molecules) is used. The present inventors thought that, unlike in the previously established method, it is unnecessary to use a large excess of the co-factor when using a nucleotide co-factor like ATPγS that is hardly hydrolyzed by a nucleoside triphosphatase activity such as the ATPase activity of RecA-like recombinase itself, and also thought that the presence of a large excess of a nonhydrolyzable nucleotide co-factor such as ATPγS can give negative effects on reaction efficiency, yield, or specificity of the formation of RecA protein/single-stranded nucleic acid probe/double-stranded target nucleic acid complex. The inventors evaluated in detail that how many times more the molecule number of the nonhydrolyzable nucleotide co-factor is than the molecule number of nucleotide residues constituting the single-stranded nucleic acid probe and than the molecule number of RecA protein should be optimal within the reaction solution. As a result, the inventors found that it is possible to form RecA-like recombinase/single-stranded nucleic acid probe/double-stranded target nucleic acid complex very efficiently and specifically, when RecA-like recombinase/single-stranded nucleic acid probe (containing a homologous probe having a sequence sufficiently complementary to the double-stranded target nucleic acid sequence) complex (single-stranded nucleoprotein filament) is prepared under conditions where the molecule number of nonhydrolyzable nucleotide co-factor is one quarter or more of the number of molecules of nucleotide residues within the single-stranded nucleic acid probe, and 10 times or less, preferably 5 times or less, more preferably 3 times or less the number of RecA-like recombinase molecules, and then contacting the complex with a sample containing the double-stranded target nucleic acid.

The present inventors also found that not only the fidelity and specificity of the reaction, but also the reaction efficiency and yield are markedly improved by the presence of a monovalent cation during the homologous pairing reaction of the double-stranded target nucleic acid using the RecA-like recombinase/single-stranded nucleic acid probe complex prepared, completing the present invention.

The present invention relates to a method for preparing a RecA-like recombinase/single-stranded nucleic acid probe (containing a homologous probe having a sequence sufficiently complementary to the double-stranded target nucleic acid sequence) complex exhibiting high reactivity and specificity to the double-stranded target nucleic acid in a sample, as well as the utilization of the RecA-like recombinase/single-stranded nucleic acid probe complex prepared by the method of the invention for targeting, enriching, detecting, and/or isolating the double-stranded target nucleic acid sequence. Specifically, the present invention provides:
(1) a method for preparing a RecA-like recombinase/single-stranded nucleic acid probe complex, the method comprising reacting a single-stranded nucleic acid probe sample containing a homologous probe with a RecA-like recombinase in the presence of a nonhydrolyzable nucleotide co-factor the number of molecules of which is one quarter or more of the number of molecules of nucleotide residues in the single-stranded nucleic acid probe and 10 times or less the number of molecules of the RecA-like recombinase;
(2) the method of (1), wherein the nonhydrolyzable nucleotide co-factor is ATPγS, ADP • AlF₄⁻ (a mixture of ATP, aluminum nitrate, and sodium fluoride, or a mixture of ADP, aluminum nitrate, and sodium fluoride), dADP • AlF₄⁻ (a mixture of dATP, aluminum nitrate, and sodium fluoride, or a mixture of dADP, aluminum nitrate, and sodium fluoride), ADP • BeF₃⁻ (a mixture of ATP, beryllium sulfate, and sodium fluoride, or a mixture of ADP, beryllium sulfate, and sodium fluoride), or dADP • BeF₃⁻ (a mixture of dATP, beryllium sulfate, and sodium fluoride, or a mixture of dADP, beryllium sulfate, and sodium fluoride);
(3) the method of (1), wherein the homologous probe is at least two types of homologous probes that are sufficiently complementary to one another;
(4) the method of any one of (1) to (3), wherein the single-stranded nucleic acid probe sample is a mixture of the homologous probe and at least one type of heterologous probe;
(5) the method of (1), wherein the single-stranded nucleic acid probe sample is reacted with the RecA-like recombinase in the presence of 0.5 to 2.0 mM magnesium ions;
(6) the method of (1), wherein the RecA-like recombinase is derived from a prokaryote;
(7) the method of (1), wherein the RecA-like recombinase is derived from *Escherichia coli;*
(8) the method of any one of (1), (6), and (7), wherein the RecA-like recombinase has a label or a ligand;
(9) the method of any one of (1) to (7), wherein the homologous probe has a label or a ligand;
(10) a kit comprising the RecA-like recombinase and the nonhydrolyzable nucleotide co-factor, the kit being used for preparing the RecA-like recombinase/single-stranded nucleic acid probe complex of any one of (1) to (9);
(11) a method for targeting, enriching, detecting, and/or isolating a double-stranded target nucleic acid in a sample, the method comprising:
   (a) contacting a RecA-like recombinase/single-stranded nucleic acid probe complex prepared by the method of (9) with a sample containing the double-stranded target nucleic acid,
   (b) trapping, onto a solid phase, a formed complex of the double-stranded target nucleic acid with the homologous probe having a label or a ligand, and
   (c) removing the double-stranded nucleic acid and the probe that are not trapped onto the solid phase;
(12) a method for targeting, enriching, detecting, and/or isolating a double-stranded target nucleic acid in a sample, the method comprising:
   (a) contacting a RecA-like recombinase/single-stranded nucleic acid probe complex prepared by the method of (9) with a sample containing the double-stranded target nucleic acid inserted into a transformation vector,
   (b) trapping, onto a solid phase, a formed complex of the double-stranded target nucleic acid with the homologous probe having a label or a ligand,
   (c) removing the double-stranded nucleic acid and the probe that are not trapped onto the solid phase,
   (d) releasing, from the solid phase, a fraction containing the double-stranded target nucleic acid trapped onto the solid phase, and transforming an appropriate host cell with the fraction, and
   (e) selecting the transformed cell carrying the double-stranded target nucleic acid;
(13) the method of any one of (8), (9), (11), and (12), wherein the label or ligand is biotin or digoxigenin;
(14) the method of (13), wherein the solid phase is magnetic beads to which avidin (streptavidin) or anti-digoxigenin antibody is bound;
(15) a method for detecting a double-stranded target nucleic acid in a fixed cell sample by in *situ* hybridization, wherein the RecA-like recombinase/single-stranded nucleic acid probe complex prepared by the method of (8) or (9) is used;
(16) a method for targeting a double-stranded target nucleic acid in a living cell sample by in *vivo* gene targeting, wherein the RecA-like recombinase/single-stranded nucleic acid probe complex prepared by the method of any one of (1) to (9) is used;
(17) the method of any one of (1), (11), (12), (15), and (16), wherein the double-stranded target nucleic acid is double-stranded target DNA;
(18) the method of any one of (11) to (16), wherein the RecA-like recombinase/single-stranded nucleic acid probe complex is reacted with a sample containing the double-stranded target nucleic acid in the presence of monovalent cations;
(19) the method of (18), wherein the monovalent cations are sodium ions or potassium ions;
(20) the method of (19), wherein the sodium ions are derived from 150 mM or less sodium chloride or 250 mM or less sodium acetate and the potassium ions are derived from 150 mM or less potassium chloride or 250 mM or less potassium acetate; and
(21) a kit for targeting, enriching, detecting, and/or isolating double-stranded target nucleic acid in a sample, the kit comprising the RecA-like recombinase/single-stranded nucleic acid probe complex prepared by the method of any one of (1) to (9).

In the present invention, the term "homologous probe" means a single-stranded nucleic acid probe sufficiently complementary to a double-stranded target nucleic acid sequence. The term "heterologous probe" means a single-stranded nucleic acid probe that does not have a sufficient complementarity to the double-stranded target nucleic acid sequence. In the present invention, when just the term "probe" is used, it indicates the homologous probe when used alone or a mixture of the homologous probe and the heterologous probe.

Also, in the present invention, the term "nonhydrolyzable nucleotide co-factor" means a nucleotide co-factor that is not easily hydrolyzed by a nucleoside triphosphatase activity such as ATPase of RecA-like recombinase itself.

Another term used herein, "double-stranded target nucleic acid" means double-stranded nucleic acid that is a target of the homologous probe. Herein, the term "double-stranded nucleic acid" means both double-stranded target nucleic acid and double-stranded nucleic acid that is not the target of the homologous probe.

A recombinase that can be used in the present invention means a group of RecA-like recombinase proteins that can catalyze in vitro homologous pairing and/or DNA-strand exchange, which is substantially equivalent to E. *coli* RecA protein. Such RecA-like recombinases have been isolated and purified from many prokaryotes and eukaryotes. Examples of such recombinases include wild type RecA proteins derived from E. *coli* (Shibata, T. et al., Method in Enzymology, 1983, 100, 197), and mutant types of the RecA proteins (e.g., RecA 803: Madiraju, M. etal., Proc. Natl. Acad. Sci. USA, 1988, 85, 6592; RecA441: Kawashima, H. et al., Mol. Gen. Genet., 1984, 193, 288; etc.); uvsX protein, T4 phage-derived analogues of the protein (Yonesaki, T. et al., Eur. J. Biochem., 1985, 148, 127); RecA proteins derived from *Bacillus subtilis* (Lovett, C. M. et al., J. Biol. Chem., 1985, 260, 3305); Rec1 protein derived from *Ustilago* (Kmiec, E. B. et al., Cell, 1982, 29, 367); RecA―like proteins derived from heat-resistant bacteria (such as *Thermus aquaticus* or *Thermus thermophilus)* (Angov, E. et al., J. Bacteriol., 1994, 176, 1405; Kato, R. et al., J. Biochem., 1993, 114, 926); and RecA-like proteins derived from yeast, mouse, and human (Shinohara, A. et al., Nature Genetics, 1993, 4, 239).

E. coli RecA proteins can be purified from *E*. *coli* by conventional methods and used (e.g., Kuramitsu, S. et al., J. Biochem., 1981, 90, 1033; Shibata, T. et al., Methods in Enzymology, 1983, 100, 197). Commercially available RecA proteins may also be used (manufactured by Boehringer Mannheim,Promega, etc.). *E*. *coli* RecA proteins are quantified based on "extinction coefficient ε^{1%}₂₈₀ = 5.9" (Craig, N. L. et al., J. Biol. Chem., 1981, 256, 8309-8044).

The double-stranded target nucleic acid used in the present invention encompasses RNA as well as DNA and cDNA (DNA/RNA hybrids, RNA regions having double-stranded structure, etc.). The length, type, shape, and such of the double-stranded target nucleic acid of the invention are not especially restricted. For example, the double-stranded target nucleic acid may be in a circular form (open-circular or closed-circular form) or a linear form. Preferably, the double-stranded target nucleic acid is a double-stranded target DNA. The double-stranded target DNA may be, but is not limited to, genomic DNA or cDNA derived fromprokaryotes and eukaryotes, DNA derived from viruses or bacteriophages, fragments of such genomic DNA or cDNA, and various kinds of DNA libraries containing such various DNA. The double-stranded target nucleic acid of the present invention may be within a solution; within fixed cells or cellular structures fixed by conventional methods using organic solvents (methanol, ethanol, etc.), acids (e.g., acetic acid), cross-linking agents (formalin, paraformaldehyde, etc.) and such; or within living cells or cellular structures that are not fixed. Examples of cells and cellular structures include bacteria, viruses, cellular organs such as nucleus and mitochondria, and chromosomes, which exist in cells, and parasites such as viruses or bacteria existing in biological samples such as blood.

The double-stranded target nucleic acid, if desired, may be labeled for detection and/or isolation using conventional methods well known in the art. The target nucleic acid may be labeled by, but not limited to, radio-isotopes (e.g., ³²P, ³⁵S, etc.), fluorescent pigments (e.g., FITC, rhodamine, etc.), enzyme labels (e.g., peroxidase, alkaline phosphatase, etc.), chemiluminescent agents (e.g., acridinium ester etc.), and various labels and ligands such as biotin or digoxigenin.

The single-stranded nucleic acid probe used in the present invention is a single-stranded nucleic acid, and generally, single-stranded DNA is employed. The shape of the probe is not restricted, allowing the use of either a circular form or a linear form. The single-stranded nucleic acid probe sample may be just a single-stranded nucleic acid probe (homologous probe) that is sufficiently complementary to the target sequence, or a mixture with a single-stranded nucleic acid probe (heterologous probe) that does not have a sufficient complementarity to the target sequence. The presence of the heterologous probe is known to improve the specificity (PCT/JP97/03019). It is necessary to optimize the amount of single-stranded nucleic acid probe used, according to the total amount of the sample nucleic acid, the amount of double-stranded target nucleic acid in the sample, etc.

The above mentioned "single-stranded nucleic acid probe (homologous probe) that is sufficiently complementary to the target sequence" used in the present invention is a single-stranded nucleic acid that comprises a sequence having at least 70% or more homology with the nucleotide sequence of the whole or part of the target sequence, and normally is a single-stranded DNA comprising the above sequence. In order to ensure nucleotide sequence specific homologous pairing (hybridization reaction) between the double-stranded target DNA and the homologous probe, it is preferable that the homologous probe generally contains a sequence that is at least 90% or more homologous to the partial or whole sequence of the double-stranded target DNA, and more preferable is a homology of 95% or more.

The homologous probe of the present invention may also be prepared by denaturing a double-stranded nucleic acid probe which is complementary to either one or both strands of the target sequence. The homologous probe may also contain an extended terminal portion that is not complementary to any of the nucleic acid strands in the sample. When both strands of the double-stranded homologous probe have such extended sequences at their termini, the extended sequences may be complementary to each other.

Many commercially available single-stranded and double-stranded nucleic acid probes are usable. Alternatively, the probe can be prepared by a probe-preparation method known to those skilled in the art. For example, the probe can be obtained directly from any one of plasmids, cosmids, or other vectors containing the sequence. If desired, the portion corresponding to the probe is removed from the vector by using restriction enzymes, and the resulting specific fragment treated with restriction enzymes is isolated by electrophoresis. Alternatively, the portion corresponding to the probe can be amplified by PCR. Although the probe thus obtained is generally double-stranded, the probe, if desired, can be denatured to a single-stranded one. Alternatively, the double-stranded probe is subcloned into a single-stranded vector such as M13 phage vector, and then used as a single-stranded probe. An alternative method for preparing a single-stranded probe is oligonucleotide synthesis. As a longer probe is required, multiple probe sub-fragments are synthesized and joined together to prepare the probe.

A sequence homologous to the target sequence, which should be contained in the above homologous probe, is at least 15 nucleotides in length, and preferably 25 to 2,000 nucleotides in length. A longer polynucleotide-probe (2,000 or more nucleotides in length) may also be used.

If desired, a homologous probe may be labeled for detection and/or isolation using conventional methods known in the art. Examples of the labels that may be used to label a homologous probe include, but are not limited to, radio isotopes (e.g., ³²P, ³⁵S, etc.), fluorescent dyes (e.g., FITC, rhodamine, etc.), enzyme labels (e.g., peroxidase, alkaline phosphatase, etc.), chemiluminescent agents (e.g., acridinium ester etc.), and various labels and ligands such as biotin or digoxigenin.

The "heterologous probe" employed in the present invention is a nucleic acid probe which is not sufficiently complementary to the target sequence, which is usually a single-stranded DNA. In a preferred embodiment, the sequence of the heterologous probe may show low complementarity to a sequence other than the target sequence, for example, a vector sequence, in which the target sequence is integrated.

There is no restriction on the shape of the probe, and thus, either a circular form or a linear form can be used. The probe may be prepared by denaturing the double-stranded heterologous probe.

For example, if the DNA sample is derived from a human, the heterologous probe used in the system may be a single-stranded nucleic acid probe derived from various microorganisms including viruses or bacteriophages, preferably, single-stranded phage DNA such as M13 or φX174, or single-stranded DNA generated from the fragment of lambda phage DNA, a single-stranded nucleic acid probe derived from eukaryotes except humans, preferably single-stranded DNA generated from DNA fragments from salmon sperm or herring sperm, etc. Mixtures of synthetic DNA having appropriate lengths and random sequences can also be used as the heterologous probe.

Usually, the heterologous probe employed in the present invention does not contain any labels or ligands. The heterologous probe used herein is at least 15 nucleotides in length. Preferably it is about 30 to 10,000 nucleotides in length, and more preferably 60 to 7,000 nucleotides in length. A longer polynucleotide probe, such as a probe of 10,000 nucleotides or more in length, may also be used.

When the probe to be used is a mixture of a homologous probe and a heterologous probe, the preferable weight ratio between them is from about 1:1 to about 1:500. It is necessary to optimize total amounts of homologous probe and heterologous probe used as well as the weight ratio between them, according to the total amount of the sample nucleic acid, the amount of double-stranded target nucleic acid in the sample, etc.

Nonhydrolyzable nucleotide co-factors usable in the present invention include ATPγS , ADP · AlF₄⁻(a mixture of ATP, aluminum nitrate, and sodium fluoride; or a mixture of ADP, aluminum nitrate, and sodium fluoride), dADP · AlF₄⁻ (a mixture of dATP, aluminum nitrate, and sodium fluoride; or a mixture of dADP, aluminum nitrate, and sodium fluoride), ADP · BeF₃⁻ (a mixture of ATP, beryllium sulfate, and sodium fluoride; or a mixture of ADP, beryllium sulfate, and sodium fluoride), and dADP · BeF₃⁻ (a mixture of dATP, beryllium sulfate, and sodium fluoride; or a mixture of dADP, beryllium sulfate, and sodium fluoride) (Moreau, L. P. et al., J. Biol. Chem., 1989, 264, 2302-2306; Lange, A. J. et al., J. Biol. Chem., 1986, 261, 101-107; Kowalczykowski, S. C. et al., Proc. Natl. Acad. Sci. USA, 1995, 92, 3478-3482).

A RecA-like recombinase/single-stranded nucleic acid probe complex (single-stranded nucleoprotein filament), which is suitable for the targeting, enrichment, detection, and/or isolation of the double-stranded target nucleic acid in a sample, is prepared by reacting a sample of single-stranded nucleic acid probe containing a homologous probe with RecA-like recombinase when the number of molecules of the above-mentioned nonhydrolyzable nucleotide co-factor is one quarter or more of the number of molecules of nucleotide residues in the single-stranded nucleic acid probe, and 10 times or less the number of RecA-like recombinase molecules, preferably 5 times or less, more preferably 3 times or less. If ATPγS is used, ADP of which amount is 3 to 4 times more than that of ATPγS can coexist with ATPγS.

Specifically, the above-mentioned homologous probe when used alone, or a mixture of a homologous probe and a heterologous probe, is usually pre-heated at about 95 to 100°C for about 5 minutes to denature it, thereby preparing a single-stranded nucleic acid probe sample. Then, the probe is cooled on ice for about 20 to 60 seconds. The resulting probe is used for the binding reaction with the above-mentioned RecA protein. If desired, the probe is centrifuged at 0 to 4°C for about 5 to 20 seconds prior to the binding reaction with RecA protein. Although the probe converted to a single strand by denaturation can be stored at -20 °C in a freezer, it is preferable to immediately mix the single-stranded probe with the nonhydrolyzable nucleotide co-factor and RecA protein at the above-mentioned concentration ratio in a standard RecA-coating reaction solution (which may be prepared to contain each constituent at a final concentration of 1 to 100 mM (preferably 10 to 35 mM) Tris-HCl or Tris-acetate buffer (pH about 7.5), 0.5 to 12.5 mM (preferably 0.5 to 2 mM) magnesium chloride or magnesium acetate, 0 to 50 mM sodium chloride or potassium chloride, or 0 to 100 mM sodium acetate or potassium acetate, 0 to 1 mM dithiothreitol, 0 to 100 mM EGTA, 0 to 50 mM spermidine, and 0 to 10 % glycerol) within iced water. Total volume of the mixture is preferably 100 µl or less, more preferably about 5 to 40 µl. The mixture is incubated at 37°C for 5 to 20 minutes, and RecA protein is allowed to bind to the single-stranded nucleic acid probe sample in order to form the RecA protein/single-stranded nucleic acid probe complex (single-stranded nucleoprotein filament).

In the above-described coating reaction solution, RecA protein should be added at a ratio of at least 1 molecule or more per 4 nucleotide residues within the single-stranded nucleic acid probe, preferably 1 molecule or more per 3 nucleotide residues. It is necessary to optimize the total amount of RecA protein according to the total amount of nucleic acid sample containing the double-stranded target nucleic acid, as well as the amount of single-stranded nucleic acid probe used.

If desired, the reaction can be conducted in the presence of single-strand binding protein (SSB), topoisomerase I, topoisomerase II, etc.

In some cases, it is also possible to simultaneously achieve the formation of the RecA protein/single-stranded nucleic acid probe complex and the homologous pairing reaction between the complex and double-stranded target nucleic acid, by adding a nucleic acid sample containing the double-stranded target nucleic acid when mixing the single-stranded nucleic acid probe, the nonhydrolyzable nucleotide co-factor, and RecA protein with the standard RecA-coating reaction solution. In this case, the reaction is preferably conducted in the presence of 4 mM or higher concentration of Mg²⁺, or alternatively, in the presence of spermidine.

The homologous probe of the present invention may be used as a RecA protein with a label or ligand/homologous probe complex, prepared by binding the homologous probe to RecA protein having various labels or ligands like those described in the reference WO95/18236. It is not preferable, however, to use a heterologous probe prepared as a complex with RecA protein having various labels or ligands.

The RecA protein/single-stranded nucleic acid probe complex prepared herein is added to the nucleic acid sample under conditions in which the double-stranded target nucleic acid is not denatured, for example, below the temperature capable of denaturing the double-stranded nucleic acid. The mixture is then incubated under conditions suitable for the homologous pairing reaction at 37°C for 5 minutes to 24 hours, preferably for 10 minutes to 2 hours, thereby successfully achieving the formation of a complex between the double-stranded target nucleic acid and the RecA protein/single-stranded nucleic acid probe complex (RecA protein/single-stranded nucleic acid probe/double-stranded target nucleic acid complex).

Reaction conditions suitable for the homologous pairing reaction (hybridization reaction) are almost the same as those for the above-mentioned RecA-coating reaction, and the reaction can be conducted in the following reaction solution. Specifically, the respective final concentrations of constituents in the reaction solution are adjusted to be within the following range: 1 to 100 mM (preferably 10 to 35 mM) Tris-HCl or Tris-acetate buffer (pH about 7.5), 4 to 25 mM (preferably 4 to 12.5mM) magnesium chloride or magnesium acetate, 0 to 150 mM sodium chloride or 0 to 250 mM sodium acetate alternatively 0 to 150 mM potassium chloride or 0 to 250 mM potassium acetate, 0 to 1 mM dithiothreitol, 0 to 100 mM EGTA, 0 to 50 mM spermidine, and 0 to 10% glycerol.

It is unnecessary to add the nucleotide co-factor again to the reaction system at the time of the homologous pairing reaction. It is necessary to optimize the amount of RecA protein/single-stranded nucleic acid probe complex to be used in the homologous pairing reaction according to the total amount of the nucleic acid sample containing the double-stranded target nucleic acid, etc. (US Patent No. 4,888,274; PCT/JP97/03019).

The presence of a monovalent cation, for example, sodium chloride or potassium chloride of 150 mM or less, or sodium acetate or potassium acetate of 250 mM or less, can improve not only the fidelity and specificity of the reaction but also the reaction efficiency and yield in the homologous pairing reaction of the double-stranded target nucleic acid using the RecA-like recombinase/single-stranded nucleic acid probe complex prepared according to the present invention. It is necessary to optimize the type and concentration of salt to be added according to the type and shape (closed circular, open circular, or linear) of the double-stranded target nucleic acid as well as the length of a sequence homologous to the target sequence of the homologous probe, etc. For example, when the double-stranded target nucleic acid is a closed circular double-stranded DNA, the presence of sodium chloride or potassium chloride of 25 to 150 mM, or sodium acetate or potassium acetate of 50 to 250 mM, can markedly improve not only the fidelity and specificity of the reaction but also the reaction efficiency and yield.

This finding is entirely new, and different from previous findings, which are observed when the preparation of RecA protein/single-stranded DNA probe complex and homologous pairing reaction with the double-stranded target DNA are simultaneously performed in the presence of a large excess of ATPγS or ATP, that the fidelity and specificity alone are improved without alteration in the yield of probe/double-stranded target DNA complex when the homologous pairing reaction is conducted in the presence of 100 mM potassium chloride (Malkov, V. A. et al., J. Mol. Biol., 1997, 271, 168-177) and that DNA-strand exchange reaction is partly inhibited by the presence of 50 mM sodium salt and almost completely inhibited by the presence of 100 mM sodium chloride or 200 mM sodium acetate (Roman, L. J. et al., Biochemistry, 1986, 25, 7375-7385). The finding herein is also entirely different from a previous finding that D-loop formation is inhibited 20% by the presence of 20 mM sodium chloride and completely inhibited by the presence of 50 mM sodium chloride (Shibata, T. et al., J. Biol. Chem., 1981, 256, 7565-7572).

The following complexes prepared by the present invention can be detected or isolated by well-known methods in the art (US Patent No. 4,888,274; WO93/05177; WO93/05178; WO95/18236; Teintze, M. et al., Biochem. Biophys. Res. Commun., 1995, 211, 804-811; PCT/JP97/03019; etc.): a complex formed by homologous pairing between a complex of RecA protein/homologous probe with various labels or ligands, or a complex of RecA protein with various labels or ligands/homologous probe and a double-stranded target nucleic acid (a complex of RecA protein/homologous probe carrying various labels or ligands/double-stranded target nucleic acid, or a complex of RecA protein carrying various labels or ligands/homologous probe/double-stranded target nucleic acid); and a complex formed between RecA protein/homologous probe complex and double-stranded target nucleic acid with various labels or ligands (a complex of RecA protein/homologous probe/double-stranded target nucleic acid carrying various labels or ligands).

An example of a preferable method for enriching, detecting, and/or isolating the double-stranded target DNA is as follows: biotin-labeled homologous probe and unlabeled heterologous probe are mixed at an appropriate ratio to prepare a single-stranded DNA probe sample. Then, the resulting probe sample is reacted with RecA protein (used at a ratio of at least 1 molecule or more per 4 nucleotide residues in the single-stranded DNA probe) and a nonhydrolyzable nucleotide co-factor such as ATPγS (at least one quarter or more of the number of molecules of nucleotide residues in the single-stranded DNA probe, and 10 times or less, preferably 5 times or less, and more preferably 3 times or less the number of RecA protein molecules) at pH 7.5 at 37°C in the presence of 0.5 to 2 mM Mg²⁺ to prepare the RecA protein/single-stranded nucleic acid probe complex. The complex is added to the nucleic acid sample containing the double-stranded target DNA, and the mixture is incubated at 37°C in the presence of 4 to 12.5 mM Mg²⁺, and 150 mM or less sodium chloride or potassium chloride, alternatively 250 mM or less sodium acetate or potassium acetate for the homologous pairing reaction. Then, the formed biotin-labeled homologous probe/double-stranded target DNA complexes are trapped by magnetic beads with immobilized streptavidin (Dynal, etc.), and unbound free double-stranded nucleic acid and probe are washed out. Subsequently, the beads with bound biotin-labeled homologous probe/double-stranded target DNA complexes are incubated in a solution containing sodium chloride at a temperature from room temperature to 85°C for about 5 to 15 minutes to release (elute) a fraction containing the double-stranded target DNA. The double-stranded target DNA in the recovered fraction is inserted into an appropriate vector, and used for transformation of an appropriate host cell. A transformed cell having the double-stranded target DNA is selected. The target DNA can be recovered from the transformed cell. When the double-stranded target DNA is originally inserted into a transformation vector, it can be used as-it-is for transformation without any additional insertion process.

The RecA-like recombinase/single-stranded nucleic acid probe complex prepared in accordance with the present invention can be applied to, for example, the cloning of a target gene comprising enriching and/or isolating the target from a mixture of cDNAs or genomic DNAs, as well as the screening of a variety of gene libraries (cDNA libraries, and genomic DNA libraries of cosmid, P1, BAC, YAC, etc.) for the target gene.

The complex of the present invention may also be used for the following methods: amplification of the target DNA sequence (US Patent No. 5,223,414, W091/17267); various gene mapping such as RecA-Assisted Restriction Endonuclease cleavage (RARE) using an oligonucleotide probe (Ferrin, L. J. et al., Nature Genetics, 1994, 6, 379; Revet, B. M. J. et al., J. Mol. Biol., 1993, 232, 779); and nucleotide sequence-specific modification (methylation, alkylation, etc.) or cleavage of the target DNA using oligonucleotide (Koob, M. et al., Nucleic Acid Res., 1992, 20, 5831; Golub, E. I. et al., Nucleic Acid Res., 1992, 20, 3121; Mikhail, A. et al., Biochemistry, 1995, 34, 13098).

Furthermore, the complex of the present invention may be used for isolation and/or detection of the specific target DNA from clinical specimens containing a mixture of cDNA, genomic DNA, etc. Therefore, the complex of the present invention allows the diagnosis of a variety of genetic aberrations or mutations, or infectious diseases caused by a variety of pathogenic microorganisms or viruses.

Moreover, the RecA-like recombinase/single-stranded nucleic acid probe complex prepared by the present invention may also be used in conjunction with an *in situ* hybridization method using the RecA protein (WO93/05177, WO95/18236). It is also applicable as a gene therapy technique and a transgenic technique through gene alternation, transcription inhibition, etc. using in *vivo* gene targeting within living cells (US Patent No. 5,468,629; WO93/22443; Golub, E. I. et al., Nucleic Acid Res., 1992, 20, 3121; Golub, E.I. et al., Proc. Natl. Acad. Sci. USA, 1993, 90, 7186; etc.).

The present invention also provides a kit for preparing the RecA-like recombinase/single-stranded nucleic acid probe complex of the present invention. Such a kit contains suitable amounts of RecA-like recombinase and the nonhydrolyzable nucleotide co-factor. It may also contain, for example, a heterologous probe.

The present invention further provides a kit for targeting, enriching, detecting, and/or isolating a double-stranded target nucleic acid in a nucleic acid sample. The kit contains the RecA-like recombinase/single-stranded nucleic acid probe complex prepared according to the present invention. Such a kit may contain, as elements other than the RecA-like recombinase/single-stranded nucleic acid probe complex, for example, a reaction termination solution, washing solution for removing unreacted double-stranded nucleic acid and single-stranded nucleic acid probe, a solid phase (for example, magnetic beads, etc.) used to trap the complex between the double-stranded target nucleic acid and a homologous probe containing a label or ligand, and/or elution buffer.

### Best Mode for Carrying out the Invention

The present invention is described below with reference to the following examples, but is not to be construed as being limited thereto.

### EXAMPLE 1: Preparation of target DNA, non-target DNA, homologous probe, and heterologous probe

### a. Preparation of double-stranded target DNA and non-target DNA

php53B, a 6.6 kb plasmid containing the complete sequence of cDNA corresponding to the sequence of human tumor suppressor gene p53 (Zakut-Houri, R. et al., EMBO J., 1985, 4, 1251) was used as circular double-stranded target DNA, and plasmid vector pUC18 (2.7 kb) was used as circular double-stranded non-target DNA. These plasmids were purified from *Escherichia coli* cells harboring each plasmid, using the QIAGEN Plasmid Maxi Kit (manufactured by QIAGEN GmbH).

### b. Preparation of the homologous probe

The double-stranded DNA fragment of 275 bp that contains the partial sequence of p53 cDNA and the 5'-end of which is biotinylated was prepared under standard DNA amplification conditions (PCR method) using Taq polymerase and the following primers: 5'-CCTTGCCGTCCCAAGCAATGGATGA-3' (SEQ ID NO: 2/ corresponding to the 1st to 25th nucleotides of nucleotide sequence of SEQ ID NO: 1) and 5'-CGTGCAAGTCACAGACTTGGCTGTC-3' (SEQ ID NO: 3/ corresponding to the 251st to 275th nucleotides of nucleotide sequence of SEQ ID N0:l). The above primers are biotinylated at their 5'-ends. The double-stranded fragment was used as a homologous probe after being denatured into single-stranded DNA by heating immediately before use. The nucleotide sequence corresponding to this region of p53 cDNA is shown as SEQ ID NO: 1.

### c. Preparation of the heterologous probe

Salmon sperm DNA (Sigma, Type III) was used as a heterologous probe after it was fragmented according to conventional methods (Maniatis, T. et al., "Molecular Cloning") and then heat-denatured into single-stranded DNA.

### EXAMPLE 2: Preparation of RecA protein/sinqle-stranded DNA probe complex (single-stranded nucleoprotein filament) in the presence of ATPγS

The double-stranded homologous probe of 275 bp prepared in EXAMPLE 1-a, which has a portion of the p53 cDNA sequence and whose 5'-end is biotinylated, and the heterologous probe prepared in EXAMPLE 1-c were mixed and diluted in either sterilized water or TE buffer (10 mM Tris-HCl, 1 mM EDTA, pH 7.5). The aliquot was transferred to a 0.6 ml microcentrifuge tube and was heated in boiling water for 5 minutes to denature the double-stranded probe. The tube was then rapidly chilled in ice water. Into the tube, 1.0 µl of 10 x coating buffer (300 mM Tris-HCl (pH 7.5 at 37°C), 20 mM MgCl₂, 4 mM DTT, 30% glycerol), 1.0 µl of ATPγS (Sigma) solution (prepared at various concentrations), and RecA protein (Boehringer-Mannheim GmbH) were added. The mixture was diluted with sterilized water to a total volume of 10 µl and was allowed to react for 12 minutes at 37°C to prepare the RecA protein/single-stranded DNA probe complex (single-stranded nucleoprotein filament) under various conditions. The conditions concerning single-stranded probe (homologous probe + heterologous probe) concentrations (molar concentration of nucleotide residues constituting a probe; 1 M = 350 g/1 ) , ATPγS concentration, RecA protein concentration, ATPyS/nucleotide residue (molar ratio), and ATPγS/RecA protein (molar ratio) are shown in detail in Table 1 below.

### EXAMPLE 3: Isolation of double-stranded target DNA using complex of RecA protein/sinqle-stranded DNA probe (target DNA : non-target DNA = 1 : 50,000)

### a. Hybridization by homologous pairing reaction

10x reaction buffer (300 mM Tris-HCl (pH 7.5 at 37°C), 20 mM MgCl₂, 4 mM DTT, 30% glycerol; 1.0 µl ), 80 mM MgCl₂ solution (1.0 µ1), php53B (circular double-stranded target DNA prepared as in Example 1-a; 49 pg), pUC18 (circular double-stranded non-target DNA; 1 µg; molar ratio of target DNA to non-target DNA is 1 : 50,000), and according to needs, NaCl, CH₃COONa, KCl, and CH₃COOK solutions (prepared to the final concentrations shown in Table 2) were mixed and diluted with sterilized water to a total volume of 10 µl. This mixture was mixed with 10 µl of the RecA protein/single-stranded DNA probe (homologous probe + heterologous probe) complex prepared in Example 2 (total volume of the reaction solution = 20 µl each) and then reacted at 37°C for 45 min in the presence of the complex. The reaction was terminated by adding a reaction termination solution containing EDTA (final concentration = about 10 mM). The conditions for preparing the RecA protein/single-stranded DNA probe complex used in each reaction and the type and concentration of salts (NaCl, CH₃COONa, KCl, and CH₃COOK) contained in the each reaction are shown in Table 2 below.

### b. Capturing/isolation by magnetic beads

The magnetic beads coated with streptavidin (manufactured by DYNAL, 20 µl) was transferred into a 0.6 ml microcentrifuge tube, and washed two times in 100 µl of 30 mM Tris-HCl and 50 mM NaCI (pH7.5) using a Magnetic Beads Separation Rack (MAGNA-SEP™). After discarding the washing solution, the whole amount of the above reaction solutions (including the reaction termination solution) was added to the microcentrifuge tube containing the washed magnetic beads, mixed well and left aside for 15 minutes at room temperature to capture the complex, while stirring every 2 to 3 minutes. The magnetic beads were then separated from the supernatant using the Magnetic Beads Separation Rack. After discarding the supernatant, magnetic beads were washed 2 to 3 times under conditions (Rigas, B. et al., Proc. Natl. Acad. Sci. USA, 1986, 83, 9591-9595; Teintze, M. et al., Biochem. Biophys. Res. Commun., 1995, 211, 804-811; etc.) that do not break the homologous probe/double-stranded target DNA complex. After discarding the washing solution, the beads were mixed well with 10 µl of 30 mM Tris-HCl and 200 mM NaCl (pH7.5), and then the mixture was heated at 85°C for 8 minutes. The supernatant containing double-stranded target DNA was recovered using a Magnetic Beads Separation Rack.

### c. Transformation

Competent cells (100 µl) prepared from E. *coli* strain JM109 according to the method of Nojima et al. (Inoue H. et al., Gene, 1990, 96, 23) was transferred into a 1.5 ml microcentrifuge tube, and mixed with 10 µl of the supernatant recovered in b. This mixture was then kept on ice for 30 minutes. The mixture was subsequently incubated for 30 seconds at 42°C, and then chilled on ice again for 1 to 2 minutes. Then, 0.5 ml of SOC medium (2% Bacto trypton, 0.5% Bacto yeast extract, 10 mM NaCl, 2.5 mM KCl, 10 mM MgCl₂, 10 mM MgSO₄, 20 mM glucose) was added to the tube and incubated at 37°C for 1 hour while shaking. The cells were spread on a LB plate containing 20 to 70 µg/ml ampicillin, 1 mg/plate 5-bromo-4-chloro-3-indolyl-β-D-galactoside (X-gal), 0.5 to 1.0 mg/plate isopropyl-β-D(-)-thiogalactopyranoside (IPTG), and incubated over night at 37°C. Under these conditions, *E. coli* transformed with php53B (double-stranded target DNA) shows white colonies, and *E. coli* transformed with pUC18 (non-target DNA) shows blue colonies.

The results of the transformation obtained under various reaction conditions are shown in Table 2.

**Table 1**

| Preparation conditions of the RecA protein/single-stranded DNA probe complex | | | | | | |
|---|---|---|---|---|---|---|
| Conditions | ATPγS concentration (*µ*M) | Single-stranded probe concentration (*µ*M) | | ATPγS/Nucleotides (Molar ratio) | RecA conc. (µM) | ATPγS/RecA (Molar ratio) |
| | | Homologous probe | Heterologous probe | | | |
| A | 6.0 | 1.0 | 29.0 | 1/5 | 6.0 | 1/1 |
| B | 6.0 | 1.0 | 29.0 | 1/5 | 12.0 | 1/2 |
| C | 7.5 | 1.0 | 29.0 | 1/4 | 7.6 | 1/1 |
| D | 7.5 | 1.0 | 29.0 | 1/4 | 15.0 | 1/2 |
| E | 10.0 | 1.0 | 29.0 | 1/3 | 10.0 | 1/1 |
| F | 15.0 | 1.0 | 29.0 | 1/2 | 15.0 | 1/1 |
| G | 20.0 | 1.0 | 29.0 | 2/3 | 10.0 | 2/1 |
| H | 50.0 | 1.0 | 29.0 | 1.67/1 | 10.0 | 5/1 |
| I | 100.0 | 1.0 | 29.0 | 3.33/1 | 10.0 | 10/1 |
| J | 300.0 | 1.0 | 29.0 | 10/1 | 10.0 | 30/1 |
| K | 500.0 | 1.0 | 29.0 | 16.7/1 | 10.0 | 50/1 |
| L | 2000 | 1.0 | 29.0 | 66.7/1 | 10.0 | 200/1 |

**Table 2**

| Results of transformation¹⁾ | | | | | | |
|---|---|---|---|---|---|---|
| No. | RecA/ probe complex preparation conditions | Type of salts | Salt (mM) | Number of white colonies (colonies) | Number of blue colonies (colonies) | Specificity (%) ²⁾ |
| 1 | C | - | 0 | 464 | 206 | 69.3 |
| 2 | E | - | 0 | 535 | 235 | 69.5 |
| 3 | G | - | 0 | 518 | 298 | 63.5 |
| 4 | H | - | 0 | 460 | 432 | 51.6 |
| 5 | I | - | 0 | 364 | 650 | 35.9 |
| 6 | J | _ | 0 | 290 | 1518 | 16.0 |
| 7 | K | - | 0 | 271 | 2390 | 10.2 |
| 8 | L | - | 0 | 222 | 3742 | 5.6 |
| 9 | A | NaCl | 100 | 130 | 214 | 37.8 |
| 10 | B | NaCl | 100 | 256 | 204 | 55.7 |
| 11 | C | NaCl | 100 | 998 | 196 | 83.6 |
| 12 | D | NaCl | 100 | 1128 | 274 | 80.5 |
| 13 | E | NaCl | 100 | 1404 | 200 | 87.5 |
| 14 | F | NaCl | 100 | 1356 | 243 | 84.8 |
| 15 | G | NaCl | 100 | 1273 | 246 | 83.8 |
| 16 | H | NaCl | 100 | 1082 | 380 | 74.0 |
| 17 | I | NaCl | 100 | 806 | 640 | 55.7 |
| 18 | J | NaCl | 100 | 641 | 1325 | 32.6 |
| 19 | K | NaCl | 100 | 601 | 1624 | 27.0 |
| 20 | L | NaCl | 100 | 594 | 1773 | 25.1 |
| 21 | E | NaCl | 25 | 722 | 295 | 71.0 |
| 22 | E | NaCl | 50 | 798 | 194 | 80.4 |
| 23 | E | NaCl | 125 | 1182 | 148 | 88.9 |
| 24 | E | NaCl | 150 | 805 | 193 | 80.7 |
| 25 | E | NaCl | 50 | 1146 | 395 | 74.4 |
| 26 | E | CH₃COONa | 100 | 1932 | 376 | 83.7 |
| 27 | E | CH₃COONa | 200 | 1418 | 298 | 82.6 |
| 28 | E | CH₃COONa | 250 | 665 | 232 | 74.1 |
| 29 | E | KCl | 25 | 769 | 239 | 76.3 |
| 30 | E | KCl | 50 | 836 | 203 | 80.5 |
| 31 | E | KCl | 100 | 1471 | 226 | 86.7 |
| 32 | E | KCl | 125 | 1162 | 220 | 84.1 |
| 33 | E | KCl | 150 | 941 | 204 | 82.2 |
| 34 | E | CH₃COOK | 50 | 987 | 258 | 79.3 |
| 35 | E | CH₃COOK | 100 | 1526 | 300 | 83.6 |
| 36 | E | CH₃COOK | 200 | 1074 | 190 | 85.0 |
| 37 | E | CH₃COOK | 250 | 706 | 196 | 78.3 |

| | | | | | | |
|---|---|---|---|---|---|---|
| 1) Mean values obtained from three repeated experiments are indicated. | | | | | | |
| 2) Specificity (%) = number of white colonies / (number of white colonies + number of blue colonies) x 100 | | | | | | |

The results of Nos. 1 to 8 and 11 to 20 clearly show that both yield and specificity of double-stranded target DNA are markedly high when double-stranded target DNA is isolated using a RecA protein/single-stranded DNA probe complex prepared under conditions (Nos. 1 to 5 and 11 to 17) in which the number of ATPγS molecules is one quarter or more of the number of molecules of nucleotide residues in the single-stranded DNA probe, and 10 times or less the number of RecA protein molecules, compared to the experiments (Nos. 6 to 8 and 18 to 20) using a RecA protein/single-stranded DNA probe complex prepared under conditions in which the amount of ATPγS was 10 times or more the number of molecules of nucleotide residues, 30 times or more the number of RecA protein molecules, or 0.5 to 2 mM, as described in US Patent No. 4,888,274. The above result was obtained regardless of the presence or absence of a salt such as sodium in the homologous pairing reaction (hybridization reaction). On the other hand, the reduction of the number of ATPγS molecules to one fifth of the number of molecules of nucleotide residues in the single-stranded DNA probe (Nos. 9 and 10) lowered the yield and specificity.

The above result shows that the RecA protein/single-stranded DNA probe complex prepared when the number of ATPγS molecules is one quarter or more of the number of molecules of nucleotide residues in the single-stranded DNA probe, and 10 times or less the number of RecA protein molecules, is most suitable for the isolation of double-stranded target DNA, giving a very high yield and specificity.

Further, comparison of the results of Nos. 1 to 8 and the results of 11, 13, and 15 to 20 shows that the presence of 100 mM sodium chloride in the homologous pairing reaction (hybridization reaction) markedly improves both yield and specificity of double-stranded target DNA.

Furthermore, the results of Nos. 13, 21 to 24 and Nos. 29 to 33 show that preferable concentrations of sodium chloride and potassium chloride to be added to the homologous pairing reaction (hybridization reaction) are both 150 mM or less. The results of Nos. 25 to 28 and Nos. 34 to 37 show that preferable concentrations of sodium acetate and potassium acetate are 250 mM or less.

The above results conclude that the presence of about 25 to 150 mM sodium chloride or potassium chloride or the presence of about 50 to 250 mM sodium acetate or potassium acetate in the homologous pairing reaction markedly improves not only the fidelity and specificity, but also the reaction efficiency and yield in the isolation of double-stranded target DNA, when the double-stranded target nucleic acid is a closed circular double-stranded DNA. This finding is entirely new, and different from previous findings, which are observed when the preparation of RecA protein/single-stranded DNA probe complex and homologous pairing reaction with the double-stranded target DNA are simultaneously performed in the presence of a large excess of ATPγS or ATP, that the fidelity and specificity alone are improved without alteration in the yield of probe/double-stranded target DNA complex when the homologous pairing reaction is conducted in the presence of 100 mM potassium chloride (Malkov, V. A. et al., J. Mol. Biol., 1997, 271, 168-177) and that DNA-strand exchange reaction is partly inhibited by the presence of 50 mM sodium salt and almost completely inhibited by the presence of 100 mM sodium chloride or 200 mM sodium acetate (Roman, L. J. et al., Biochemistry, 1986, 25, 7375-7385). The finding herein is also entirely different from a previous finding that D-loop formation is inhibited 20% by the presence of 20 mM sodium chloride and completely inhibited by the presence of 50 mM sodium chloride (Shibata, T. et al., J. Biol. Chem., 1981, 256, 7565-7572).

### Industrial Applicability

The present invention enables the preparation of a RecA-like recombinase/single-stranded nucleic acid probe (containing a homologous probe having a sequence sufficiently complementary to a double-stranded target nucleic acid sequence) complex, which is highly reactive and specific to double-stranded target nucleic acid in a sample. The use of the RecA-like recombinase/single-stranded nucleic acid probe complex prepared by the present invention enables one to efficiently target, enrich, detect, and/or isolate a double-stranded target nucleic acid sequence. Thus, the inventive method can be applied in the diagnosis of a variety of gene abnormalities and mutations, various pathogenic microorganism and virus infections, gene therapy, and transgenic techniques.

## Claims

1. A method of preparing a RecA-like recombinase/single-stranded nucleic acid probe complex, the method comprising reacting a single-stranded nucleic acid probe sample containing a homologous probe with a RecA-like recombinase in the presence of a nonhydrolyzable nucleotide co-factor the number of molecules of which is one quarter or more of the number of molecules of nucleotide residues in the single-stranded nucleic acid probe and 10 times or less the number of molecules of the RecA-like recombinase.

2. The method of claim 1, wherein the nonhydrolyzable nucleotide co-factor is ATPγS, ADP •AlF₄⁻ (a mixture of ATP, aluminum nitrate, and sodium fluoride, or a mixture of ADP, aluminum nitrate, and sodium fluoride), dADP•AlF₄⁻ (a mixture of dATP, aluminum nitrate, and sodium fluoride, or a mixture of dADP, aluminum nitrate, and sodium fluoride), ADP•BeF₃⁻ (a mixture of ATP, beryllium sulfate, and sodium fluoride, or a mixture of ADP, beryllium sulfate, and sodium fluoride), or dADP • BeF₃⁻ (a mixture of dATP, beryllium sulfate, and sodium fluoride, or a mixture of dADP, beryllium sulfate, and sodium fluoride).

3. The method of claim 1, wherein the homologous probe is at least two types of homologous probes that are sufficiently complementary to one another.

4. The method of any one of claims 1 to 3, wherein the single-stranded nucleic acid probe sample is a mixture of the homologous probe and at least one type of heterologous probe.

5. The method of claim 1, wherein the single-stranded nucleic acid probe sample is reacted with the RecA-like recombinase in the presence of 0.5 to 2.0 mM magnesium ions.

6. The method of claim 1, wherein the RecA-like recombinase is derived from a prokaryote.

7. The method of claim 1, wherein the RecA-like recombinase is derived from *Escherichia coli.*

8. The method of any one of claims 1, 6, and 7, wherein the RecA-like recombinase has a label or a ligand.

9. The method of any one of claims 1 to 7, wherein the homologous probe has a label or a ligand.

10. A kit comprising the RecA-like recombinase and the nonhydrolyzable nucleotide co-factor, the kit being used for preparing the RecA-like recombinase/single-stranded nucleic acid probe complex of any one of claims 1 to 9.

11. A method for targeting, enriching, detecting, and/or isolating a double-stranded target nucleic acid in a sample, the method comprising:
(a) contacting a RecA-like recombinase/single―stranded nucleic acid probe complex prepared by the method of claim 9 with a sample containing the double-stranded target nucleic acid;
(b) trapping, onto a solid phase, a formed complex of the double-stranded target nucleic acid with the homologous probe having a label or a ligand; and
(c) removing the double-stranded nucleic acid and the probe that are not trapped onto the solid phase.

12. A method for targeting, enriching, detecting, and/or isolating a double-stranded target nucleic acid in a sample, the method comprising:
(a) contacting a RecA-like recombinase/single-stranded nucleic acid probe complex prepared by the method of claim 9 with a sample containing the double-stranded target nucleic acid inserted into a transformation vector;
(b) trapping, onto a solid phase, a formed complex of the double-stranded target nucleic acid with the homologous probe having a label or a ligand;
(c) removing the double-stranded nucleic acid and the probe that are not trapped onto the solid phase;
(d) releasing, from the solid phase, a fraction containing the double-stranded target nucleic acid trapped onto the solid phase, and transforming an appropriate host cell with the fraction; and
(e) selecting the transformed cell carrying the double-stranded target nucleic acid.

13. The method of any one of claims 8, 9, 11, and 12, wherein the label or ligand is biotin or digoxigenin.

14. The method of claim 13, wherein the solid phase is magnetic beads to which avidin (streptavidin) or anti-digoxigenin antibody is bound.

15. A method for detecting a double-stranded target nucleic acid in a fixed cell sample by in situ hybridization, wherein the RecA-like recombinase/single-stranded nucleic acid probe complex prepared by the method of claim 8 or 9 is used.

16. A method for targeting a double-stranded target nucleic acid in a living cell sample by in *vivo* gene targeting, wherein the RecA-like recombinase/single-stranded nucleic acid probe complex prepared by the method of any one of claims 1 to 9 is used.

17. The methodof any one of claims 1, 11, 12, 15, and 16, wherein the double-stranded target nucleic acid is double-stranded target DNA.

18. The method of any one of claims 11 to 16, wherein the RecA-like recombinase/single-stranded nucleic acid probe complex is reacted with a sample containing the double-stranded target nucleic acid in the presence of monovalent cations.

19. The method of claim 18, wherein the monovalent cations are sodium ions or potassium ions.

20. The method of claim 19, wherein the sodium ions are derived from 150 mM or less sodium chloride or 250 mM or less sodium acetate and the potassium ions are derived from 150 mM or less potassium chloride or 250 mM or less potassium acetate.

21. A kit for targeting, enriching, detecting, and/or isolating double-stranded target nucleic acid in a sample, the kit comprising the RecA-like recombinase/single-stranded nucleic acid probe complex prepared by a method of any one of claims 1 to 9.


SEQUENCE LISTING

<110> DAIKIN INDUSTRIES, LTD.

<120> Method for preparing high performance RecA-like
recombinase/single-stranded nucleic acid probe complex
and utilization thereof

<130> D2-001PCT

<150> JP 1998-280380
<151> 1998-10-01

<160> 3

<170> PatentIn version 2.0

<210> 1
<211> 275
<212> DNA
<213> Homo sapiens

<400> 1

ccttgccgtc ccaagcaatg gatgatttga tgctgtcccc ggacgatatt gaacaatggt 60
tcactgaaga cccaggtcca gatgaagctc ccagaatgcc agaggctgct ccccgcgtgg 120
cccctgcacc agcagctcct acaccggcgg cccctgcacc agccccctcc tggcccctgt 180
catcttctgt cccttcccag aaaacctacc agggcagcta cggtttccgt ctgggcttct 240
tgcattctgg gacagccaag tctgtgactt gcacg 275

<210> 2
<211> 25
<212> DNA
<213> Artificial Sequence

<220>
<223> Description of Artificial Sequence: Artificially Synthesized Primer
Sequence

<400> 2
ccttgccgtc ccaagcaatg gatga 25

<210> 3
<211> 25
<212> DNA
<213> Artificial Sequence

<220>
<223> Description of Artificial Sequence: Artificially Synthesized Primer
Sequence

<400> 3

cgtgcaagtc acagacttgg ctgtc 25